# EUROPEAN PATENT SPECIFICATION

(11) **EP 0 788 344 B1**
(45) Date of publication and mention of the grant of the patent: **04.10.2000**
(21) Application number: 95918819.4
(22) Date of filing: 05.05.1995
(51) Int. Cl.: A61K 6/087

(54) **PHOTOPOLYMERIZABLE COMPOSITION**
PHOTOPOLYMERISIERBARE ZUSAMMENSETZUNG
COMPOSITION PHOTOPOLYMERISABLE

(30) Priority: 05.05.1994 SE 9401558
(43) Date of publication of application: 13.08.1997
(73) Proprietor: ORALEG AB, 131 50 Saltsjö-Duvnäs (SE)
(72) Inventor: WICTORIN, Lennart, S-131 50 Saltsjö-Duvnäs (SE); LARSSON, Anders, S-161 49 Bromma (SE); JÖNSSON, Sonny, E, S- 103 39 Stockholm (SE)
(74) Representative: Fagerlin, Heléne
(86) International application number: SE9500494
(87) International publication number: WO9530402

(56) References cited:
- EP-A- 0 002 831
- EP-A- 0 330 117

## Description

The present invention relates to a photopolymerizable composition which is intended primarily for use as dental filling material. Shrinkage of the inventive composition when hardening is markedly reduced in relation to the shrinkage of known photopolymerizable dental filling compositions.

Photopolymerizable dental filling compositions have long been known. EP-A- 0 002 831 describes some such compositions that contain photopolymerizable esters, such as diacrylates or dimethacrylates. The compositions can be hardened in a dental cavity by the influence of light in the presence of given polymerization initiators. The light used in this regard is UV-light (320-400 nm) and visible light in the wavelength range of 400-500 nm.

A common problem with the polymers hitherto used in this regard is that they shrink during the polymerization process, also referred to as hardening shrinkage. All of the polymers in plastic composites at present used as dental filling material shrink during the hardening process. This hardening shrinkage can vary between 2 and 10 percent by volume, depending on the type of polymer concerned and the amount of filling material used.

This shrinkage results in uncontrollable stresses in the composite material, and also leads to the successive development of a gap between the dental filling and the tooth. This gap can be the cause of bacterial growth, the plaque agglomeration and the onset of secondary caries.

Several attempts have been made to compensate for the shrinkage of the polymer in the hardening process, for instance by etching the enamel edge of the tooth with 40% phosphoric acid, to enable the polymer to fasten mechanically to the porous enamel layer engendered by the etching process, or by mixing-in a large amount of filling material to reduce the aforesaid hardening shrinkage of the polymer.

However, these measures have only compensated for shrinkage and associated unfavourable properties to a limited extent. The requisite contents of filling material result in high viscosity composites, which result in other unsuitable properties.

Certain polymers harden as the ring opens, wherewith no hardening shrinkage occurs, or occurs only to a small extent. Epoxy polymers and spiroorthocarbonates are examples of such polymers.

Spiroorthocarbonates have been proposed as non-shrinking dental material (Thompson, et al, "Dental resins with reduced shrinkage during hardening", J. Dent. Res. 58:1522 (1979)). However, due to the crystalline character of the monomer, it is difficult to formulate from the monomers material that has suitable application properties. Spiroorthocarbonates are also difficult to synthesize and demand a very high price.

Liquid epoxides are easy to produce and a large number of such products are commercially available. However, the products are normally polymerized by adding diamines, which are toxic and allergenic and therewith not suitable for dental use.

However, it has been found that certain epoxides can be polymerized with the aid of photoinitiators when using UV-light or visible light. These epoxides were found to have low index values when subjected to skin irritation tests (0.5 in an index scale from 0 to 8). Seen from a biological point of view, these epoxides are therefore excellent as dental filling material, surprisingly enough.

The invention thus relates to dental compositions which include compounds according to the general formula (I) 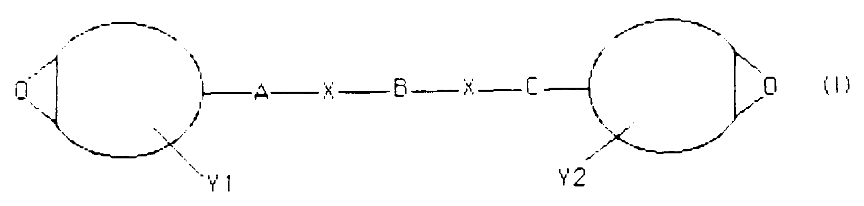 where
A, B and C are the same or different and denote a straight or branched, substituted or non-substituted carbon chain having 0 to 10 carbon atoms, preferably 0 to 2 carbon atoms; and
X may be -0-, -NHCO-, -NH-, -CO- or a chemical bond; and
Y1 and Y2 are the same or different and may be a substituted or non-substituted carbocyclic or heterocyclic, aliphatic or aromatic ring with 5, 6 or 7 atoms in the ring.

Both Y1 and Y2 preferably denote aliphatic rings having 6 carbon atoms, and the compound 3,4-epoxycyclohexyl-methyl-3,4-epoxy-cyclohexane-carboxylate (formula II) is thus particularly preferred.

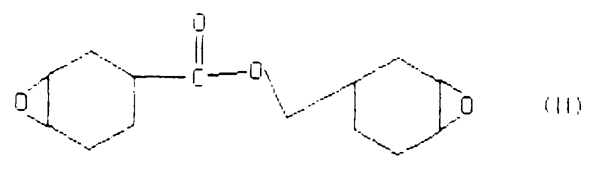

According to a first alternative, these epoxy monomers are cross-linked with caprolactone-triol (formula III). The cross-linkers are used in amounts typical in this field. According to a second alternative, the composition does not undergo cross-linking.

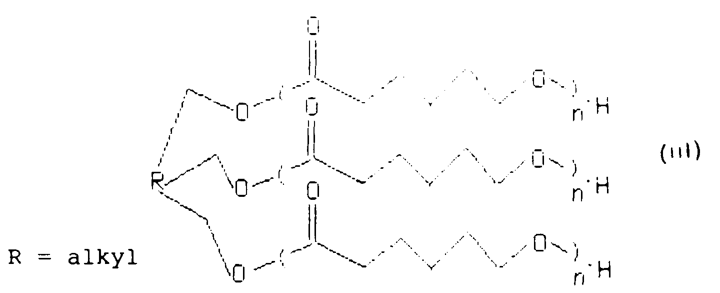

According to a first alternative, the composition is polymerized in the presence of a cation initiator, such as mixed triaryl sulfone salts and hexafluorophosphate salts (formulae IV and V), and UV-light. The photoinitiator undergoes photolysis when irradiated with UV-light in the wavelength range of 320-400 nm, which initiates polymerization of the epoxide.

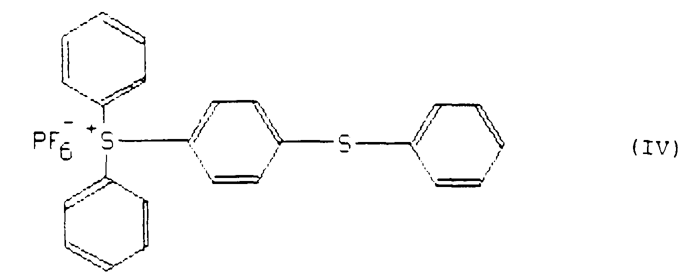

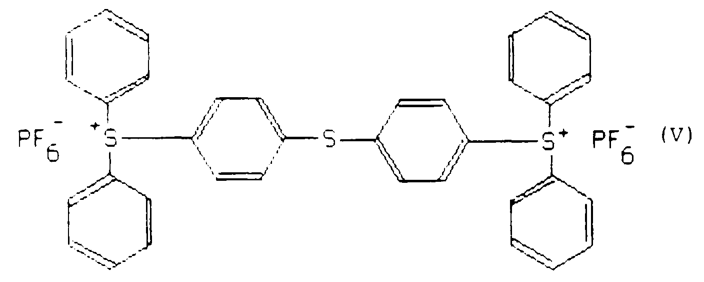

According to a second alternative, the composition is polymerized in the presence of a ferrocene-type cation initiator (formula VI) and visible light in the wavelength range of 400-500 nm. This photoinitiator is particularly useful with pigment polymers and has particular hardening characteristics in the longer wavelength range, so as to be able to harden thicker polymer layers (1-2.5 mm).

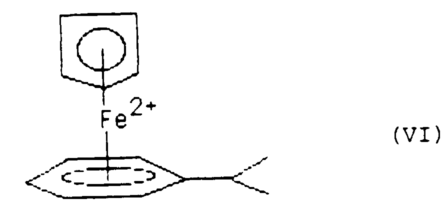

The compositions are particularly directed to use in dental care, such as for dental fillings, dental cement, fissure sealing, among other things.

The inventive compositions may also contain up to 80 percent by weight filler. The fillers that are normally used by one skilled in this art, such as quartz, fiberglass and hydroxylapatite, for instance, can also be used in the inventive compositions. Special effects can be achieved with the use of a filler, for instance barium glass, such as an aesthetic colouring effect and X-ray contrast effect, and altered mechanical properties which enhance wear resistance, for instance, and flexural strength. The fillers are also able to contribute towards further reduction in hardening shrinkage. Various fillers can be used to obtain different types of composite properties. It is known that pyrogenic silica can provide a desirable viscosity, and that fluorine-containing components can provide a suitable therapeutic effect (Transactions of Second International Congress on Dental Materials, Nov. 1-3, 1993, Hawaii, U.S.A.).

The viscosity of the composition can also be increased by adding a thickening agent. Examples of such thickening agents are co-polymers of vinyl acetate, vinyl chloride and/or vinyl alcohol. Thickening agents in powder form are dissolved in the epoxide while applying heat.

The viscosity of the composition is increased in this way to provide a composition consistency that is suitable for insertion into a tooth cavity. The consistency of the composition can therewith be adapted to the technique applied, such that the composition is able to spread out to conform to the shape of the cavity and to adhere to the cavity walls.

Calcium fluoride is highly permeable to light and therefore affords a deeper hardening depth. Normally, a plastic composite which contains more than 50% filler cannot be hardened to a depth greater than 2 mm. If the cavity is deeper than 2 mm, the hardening process must be carried out in stages. When the composition contains less filler, for instance from 10-30%, the requirement with regard to the light permeability of the composite is not equally as high. This enables the use of fillers that are more suitable from a biological, chemical and physical aspect to be used.

Since there is no hardening shrinkage, or very little hardening shrinkage, with a composition according to the invention, there is no need to include large quantities of filler (more than 50%) in order to compensate for shrinkage of the polymer as it hardens.

Hydroxylapatite (HA) is present as the inorganic component in dental substance and tissue. It is preferred to use HA as a filler in the inventive compositions, primarily because of its good biological properties.

The invention will now be described with reference to a number of examples, which have no limiting significance.

### Example 1

A mixture containing 80% by volume 3,4-epoxycyclohexylmethyl- 3,4-epoxy-cyclohexane-carboxylate (monomer) (Union Carbide), 16% by volume ε-caprolactone-triol (cross-linker) (Union Carbide) and 4% by volume mixed triaryl-sulfone-hexafluorophosphate salts (Union Carbide) (initiators for UV-light, see formulae III and IV) was thoroughly stirred and hardened with UV-light in the wavelength range of 320-400 nm. A polymer having acceptable properties for use as dental filling material was obtained within the space of 1-2 minutes, the length of time required depending on the intensity of the hardening source.

### Example 2

A mixture containing 80% by volume bis-(3,4-epoxycyclohexyl)-adipate (monomer) (Union Carbide), 16% by volume e-caprolactone-triol (cross-linker) (Union Carbide) and 4% by volume of mixed triaryl-sulfone-hexafluorophosphate salts (Union Carbide) (initiators for UV-light, see formulae III and IV) was thoroughly stirred and hardened with the aid of UV-light in the wavelength range of 320-400 nm. A polymer having acceptable properties for use as dental filling material was obtained within 1-2 minutes, the length of time required depending on the intensity of the hardening source.

### Example 3

A plastic compound containing 78% by volume 3,4-epoxycyclohexyl-methyl- 3,4-epoxy-cyclohexane-carboxylate, 14% by volume ε-caprolactone-triol, 4% by volume mixed triaryl-sulfone-hexafluorophosphate salts and 4% by volume VAGH (Vinyl acetate, vinyl chloride and vinyl alcohol (thickening agent)) was thoroughly stirred and hardened with UV-light in the wavelength range of 320-400 nm. A polymer having acceptable properties for use as dental filling material was obtained within 1-2 minutes.

### Example 4

A plastic compound containing 78% by volume bis-(3,4-epoxy-cyclohexyl)-adipate, 14% by volume ε-caprolactone-triol, 4% by volume mixed triaryl-sulfone-hexafluorophosphate salts and 4% by volume VAGH (Vinyl acetate, vinyl chloride and vinyl alcohol (thickening agent) was thoroughly stirred and hardened with UV-light in the wavelength range of 320-400 nm. A polymer having acceptable properties for use as dental filling material was obtained within 1-2 minutes.

### Example 5

A plastic compound containing 58.5% by volume 3,4-epoxycyclohexyl-methyl-3,4-epoxycyclohexyl-carboxylate, 10.5% by volume ε-caprolactone-triol, 3% by volume mixed triaryl-sulfone-hexafluorophosphate salts, 3% by volume VAGH and 25% by volume of coloured quartz (filler) was thoroughly stirred and hardened with UV-light in the wavelength range of 320-400 nm. A polymer having acceptable properties for use as a dental filling material was obtained within 1-2 minutes.

### Example 6

A plastic compound containing 58.5% by volume bis-(3,4-epoxycyclohexyl)-adipate, 10,5% by volume ε-caprolactone-triol, 3% by volume mixed triaryl-sulfone-hexafluorophosphate salts, 3% by volume VAGH and 25% by volume of coloured quartz (filler) was thoroughly stirred and hardened with UV-light in the wavelength range of 320-400 nm. A polymer having acceptable properties for use as dental filling material was obtained within 1-2 minutes.

### Example 7

A plastic compound containing 40% by volume 3,4-epoxycyclohexyl methyl-3,4-epoxycyclohexyl-carboxylate, 8% by volume ε-caprolactone-triol, 2% by volume mixed triaryl-sulfone-hexafluorophosphate salts and 50% by volume finely-ground fiberglass (0.1 mm) (filler) was thoroughly stirred and hardened with UV-light in the wavelength range of 320-400 nm. A polymer having acceptable properties for use as dental filling material was obtained within 1-2 minutes.

### Example 8

A plastic compound containing 40% by volume bis-(3,4-epoxycyclohexyl)-adipate, 8% by volume e-caprolactone-triol, 2% by volume mixed triaryl-sulfone-hexafluorophosphate salts and 50% by volume finely-ground fiberglass (0.1 mm) (filler) was thoroughly stirred and hardened with UV-light in the wavelength range of 320-400 nm. A polymer having acceptable properties for use as dental filling material was obtained within 1-2 minutes.

### Example 9

A plastic compound containing 91% by volume 3,4-epoxycyclohexyl-methyl-3,4-epoxy-cyclohexane-carboxylate, 9% by volume (η⁵-2,4-cyclopentadiene-1-yl) [1,2,3,4,5,6-η)-(1-methylethyl)-benzene]-iron-hexafluorophosphate (initiator for visible light) was thoroughly stirred and hardened with light in the wavelength range of 400-500 nm. A polymer having acceptable properties for use as dental filling material was obtained within 1-2 minutes.

The compound can be supplemented with a suitable filler, such as fiberglass, quartz and coloured calcium fluoride adapted to an appropriate consistency.

### Example 10

A plastic compound containing 91% by volume bis-(3,4-epoxycyclohexyl)-adipate, 9% by volume (η⁵-2,4-cyclopentadiene-1-yl) [1,2,3,4,5,6-η)-1-methylethyl)-benzene]-iron-hexafluorophosphate (initiator for visible light) was thoroughly stirred and hardened with light in the wavelength range of 400-500 nm. A polymer having acceptable properties for use as dental filling material was obtained within 1-2 minutes.

The compound can be supplemented with a suitable filler, such as fiberglass, quartz and coloured calcium fluoride adapted to an appropriate consistency.

### Example 11

A plastic compound containing 80% by volume 3,4-epoxycyclohexyl-methyl-3,4-epoxy-cyclohexane-carboxylate, 16% by volume ε-caprolactone-triol and 4% by volume (η⁵-2,4-cyclopentadiene-1-yl)[1,2,3,4,5,6-η)-(1-methylethyl)-benzene]-iron-hexafluorophosphate was thoroughly stirred and hardened with light in the wavelength range of 400-500 nm. A polymer having acceptable properties for use as dental filling material was obtained within 1-2 minutes.

The compound can be supplemented with a suitable filler, such as fiberglass, quartz and coloured calcium fluoride adapted to an appropriate consistency.

### Example 12

A plastic compound containing 80% by volume bis-(3,4-epoxycyclohexyl)-adipate, 16% by volume ε-caprolactone-triol and 4% by volume (η⁵-2,4-cyclopentadiene-1-yl) [1,2,3,4,5,6-η)-(1-methylethyl)-benzene]-iron-hexafluorophosphate was thoroughly stirred and hardened with light in the wavelength range of 400-500 nm. A polymer having acceptable properties for use as dental filling material was obtained within 1-2 minutes.

The compound can be supplemented with a suitable filler, such as fiberglass, quartz and coloured calcium fluoride adapted to an appropriate consistency.

### Example 13

A plastic compound containing 48% by volume 3,4-epoxycyclohexyl-methyl-3,4-epoxy-cyclohexane-carboxylate, 48% by volume bis-(3,4-epoxycyclohexyl)-adipate and 4% by volume (η5-2,4-cyclopentadiene-1-yl)[(1,2,3,4,5,6-η)-(1-methylethyl)-benzene]-iron-hexafluorophosphate was thoroughly stirred and hardened with light in the wavelength range of 400-500 nm. A polymer having acceptable properties for use as dental filling material was obtained within 1-2 minutes.

The compound can be supplemented with a suitable filler, such as fiberglass, quartz and coloured calcium fluoride adapted to an appropriate consistency.

### Example 14

A plastic compound containing 41% by volume 3,4-epoxycyclohexyl-methyl-3,4-epoxy-cyclohexane-carboxylate, 41% by volume bis-(3,4-epoxycyclohexyl)-adipate, 16% by volume ε-caprolactone-triol and 2% by volume mixed triaryl sulfone-hexafluorophosphate salts was thoroughly stirred and hardened with light in the wavelength of range 320-400 nm. A polymer having acceptable properties for use as dental filling material was obtained within 1-2 minutes.

The compound can be supplemented with a suitable filler, such as fiberglass, quartz and coloured calcium fluoride adapted to an appropriate consistency.

### Example 15

It was possible to calculate the linear shrinkage in conjunction with hardening, by producing sample bodies in a specially produced steel mould. Sample bodies measuring 25 x 2.5 mm were produced from the compositions recited in Examples 2 and 3, by hardening the compositions in the steel moulds with UV-light in the wavelength range of 320-400 nm. The linear shrinkage was then determined in accordance with standard ASTM from D 2566-79. The values obtained are shown in Table 1 below. The standard method used is intended for heat-hardening moulded plastics during polymerization.

### Example 16

The flexural strength of the compositions described in Examples 3 and 5 was investigated. Sample bodies were produced in accordance with ISO Standard No. 4049 and hardened with UV-light in the wavelength range of 320-400 nm. The results are set forth in Table 1 and show that the compositions fulfil the ISO standard, which is 50 MPa. The value is also higher than that of normally used dental composites, which have a flexural strength of about 80 MPa.

**Table 1**

| **Flexural Strength and Linear Shrinkage** | | |
|---|---|---|
| Material | Flexural strength (MPa) | Linear shrinkage (%) |
| According to Example 2 | 122.96 | 0.17 |
| According to Example 3 | 105.31 | 0.08 |

## Claims

1. A photopolymerizable composition for use in dental fillings, **characterized in** that the composition comprises:
a) the epoxy monomer 3,4-epoxycyclohexyl-methyl-3,4-epoxy-cyclohexane-carboxylate;
b) the cation initiator (η⁵-2,4-cycolopentadiene-1-yl)[(1,2,3,4,5,6-η)-(1-methylethyl)-benzene]-iron-hexaflourophosphate.

2. A photopolymerizable composition according to Claim 1, **characterized in** that the composition also includes the cross-linker ε-caprolactone-triol according to formula (III).

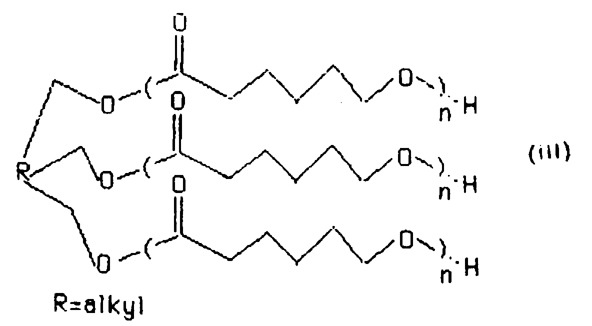

3. A photopolymerizable composition according to any one of the preceding Claims, **characterized in** that the composition includes a thickener.

4. A photopolymerizable composition according to any one of the preceding Claims, **characterized in** that the composition includes a filler.

5. A photopolymerizable composition according to Claim 4, **characterized in** that the filler is fiberglass and/or quartz.

6. A photopolymerizable composition according to any one of the preceding Claims, **characterized in** that the composition contains 25-99% by volume 3,4-epoxycyclohexyl-methyl-3,4-epoxy-cyclohexane-carboxylate and 1-75% by volume (η⁵-2,4-cyclo-pentadiene-1-yl)[(1,2,3,4,5,6-η)-(1-methylethyl)-benzene]-iron-hexafluorophosphate.

7. A photopolymerizable composition according to Claim 6, **characterized in** that the composition also includes 1-30% by volume e-caprolactone-triol, 0-10% by volume thickening agent and 0-70% by volume filler.

## Patentansprüche

1. Photopolymerisierbare Zusammensetzung zur Verwendung für Zahnfüllungen, dadurch gekennzeichnet, dass die Zusammensetzung umfasst:
a) das Epoxymonomer 3,4-Epoxycyclohexyl-methyl-3,4-epoxy-cyclohexan-carboxylat,
b) den kationischen Initiator (η⁵-2,4-Cyclopentadien-1-yl) [(1,2,3,4,5,6-η)-(1-methylethyl)-benzol]-Eisenhexafluorphosphat.

2. Photopolymerisierbare Zusammensetzung nach Anspruch 1, dadurch gekennzeichnet, dass die Zusammensetzung des weiteren den Kreuzvernetzer s-Caprolacton-triol gemäss Formel (III) beinhaltet:

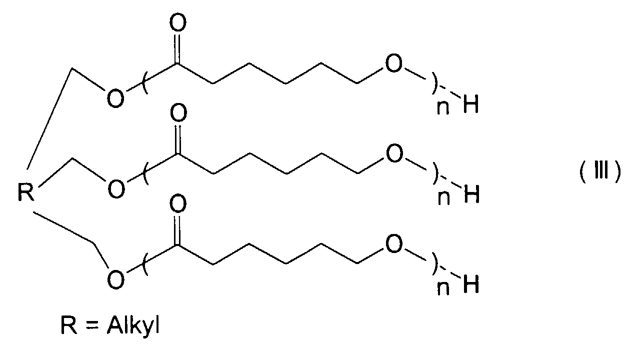

3. Photopolymerisierbare Zusammensetzung nach einem der vorherigen Ansprüche, dadurch gekennzeichnet, dass die Zusammensetzung ein Verdickungsmittel beinhaltet.

4. Photopolymerisierbare Zusammensetzung nach einem der vorherigen Ansprüche, dadurch gekennzeichnet, dass die Zusammensetzung ein Füllmittel beinhaltet.

5. Photopolymerisierbare Zusammensetzung nach Anspruch 4, dadurch gekennzeichnet, dass das Füllmittel Fiberglas und/oder Quarz ist.

6. Photopolymerisierbare Zusammensetzung nach einem der vorherigen Ansprüche, dadurch gekennzeichnet, dass die Zusammensetzung 25-99 Vol.-% 3,4-Epoxycyclohexyl-methyl-3,4-epoxy-cyclohexan-carboxylat und 1-75 Vol.-% (η⁵-2,4-Cyclopentadien-1-yl) [(1,2,3,4,5,6-η)-(1-methylethyl)-benzol]-Eisen-hexafluorphosphat enthält.

7. Photopolymerisierbare Zusammensetzung nach Anspruch 6, dadurch gekennzeichnet, dass die Zusammensetzung des weiteren 1-30 Vol.-% s-Caprolacton-triol, 0-10 Vol.-% Verdickungsmittel und 0-70 Vol.-% Füllmittel enthält.

## Revendications

1. Composition photopolymérisable utilisable pour le plombage dentaire, caractérisée en ce que ladite composition comprend :
a) le monomère époxy 3,4-époxy-cyclohexane carboxylate de 3,4-époxycyclohexylméthyle;
b) le cation initiateur de l'hexafluorophosphate de (η⁵-2,4-cyclopentadiène-1-yl)[(1,2,3,4,5,6-η)-(1-méthyléthyl)-benzène]-fer.

2. Composition photopolymérisable selon la revendication 1, caractérisée en ce que ladite composition comprend en outre un agent de réticulation de type ε-caprolactone-triol de formule (III) 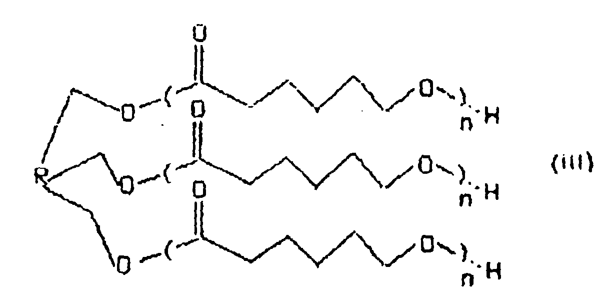 dans laquelle R est alkyle.

3. Composition photopolymérisable selon l'une quelconque des revendications précédentes, caractérisée en ce que ladite composition comprend un agent épaississant.

4. Composition photopolymérisable selon l'une quelconque des revendications précédentes, caractérisée en ce que ladite composition comprend une charge.

5. Composition photopolymérisable selon la revendication 4, caractérisée en ce que la charge est une fibre de verre et/ou du quartz.

6. Composition photopolymérisable selon l'une quelconque des revendications précédentes, caractérisée en ce que la composition comprend de 25-99 % en volume du 3,4-époxy-cyclohexanecarboxylate de 3,4-époxycyclohexylméthyl et de 1-75 % en volume de l'hexafluorophosphate de (η⁵-2,4-cyclopentadiène-1-yl)[(1,2,3,4,5,6-η)-(1-méthyléthyl)-benzène]-fer.

7. Composition photopolymérisable selon la revendication 6, caractérisée en ce que ladite composition comprend également 1 à 30 % en volume de l'ε-caprolactone-triol, de 0 à 10 % en volume de l'agent épaississant et de 0 à 70 % en volume d'une charge.
